(19) Europäisches Patentamt
European Patent Office
Office européen des brevets

(11) Numéro de publication: 0 141 732
B1

(12) FASCICULE DE BREVET EUROPEEN

(45) Date de publication du fascicule du brevet: 11.05.88

(51) Int. Cl.$^4$: A 61 K 7/50

(21) Numéro de dépôt: 84402144.4

(22) Date de dépôt: 24.10.84

# (54) Composition cosmetique de nettoyage en particulier pour le démaquillage des yeux.

(30) Priorité: 25.10.83 FR 8316978

(43) Date de publication de la demande:
15.05.85 Bulletin 85/20

(45) Mention de la délivrance du brevet:
11.05.88 Bulletin 88/19

(84) Etats contractants désignés:
BE CH DE FR GB IT LI

(56) Documents cités:
GB-A-2 128 627

(73) Titulaire: L'OREAL
14, Rue Royale
F-75008 Paris (FR)

(72) Inventeur: Contamin, Jean-Claude
14, avenue du Château
F-91420 Morangis (FR)

(74) Mandataire: Nony, Michel et al
Cabinet Nony 29, rue Cambacérès
F-75008 Paris (FR)

EP 0 141 732 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention a pour objet une nouvelle composition cosmétique de nettoyage, notamment une lotion de démaquillage des yeux, présentant un excellent pouvoir démaquillant, une bonne innocuité en test oculaire, un bon confort cosmétique et une excellente conservation non seulement dans le temps mais à différentes températures de stockage.

Il a déjà été proposé dans les brevets français No. 77.15292, 77.20116, 77.39840 et dans le certificat d'addition No. 79.14693 (Certificat d'addition au brevet français No. 77.20116) diverses lotions démaquillantes pour les yeux dont l'agent tensio-actif était essentiellement constitué par un alcoyl ou hydroxyalcoyl polyglycoside dont le radical alcoyle a de 11 à 18 atomes de carbone et dont de nombre de motifs glycosides est compris entre 3 et 25; du monolaurate ou du mono-oléate de sorbitan oxyéthyléné à l'aide de 20 moles d'oxyde d'éthylène; un alcoyle carboxylate d'α-méthyl glucoside polyéthoxylé dont le radical alcoyle, linéaire ou ramifié, saturé ou insaturé, a de 12 à 22 atomes de carbone; ou un ester d'un acide gras et de glycérol polyoxyéthyléné, ledit acide gras saturé ou insaturé ayant de 12 à 18 atomes de carbone et ledit ester ayant une HLB comprise entre 15 et 18.

Ces lotions à base de ces agents tensio-actifs, bien que présentant de bonnes propriétés démaquillantes et n'entraînant aucune gêne oculaire, c'est à dire d'irritation ou de picotement des yeux, se sont néanmoins avérées, d'une part difficiles à conserver dans le temps lorsque les variations de température sont importantes et d'autre part présenter un confort cosmétique peu satisfaisant pour ce type de produit, les lotions à base de ces agents tensio-actifs ayant parfois tendance à coller les cils et paupières des utilisatrices.

Ces compositions doivent en effet pouvoir être utilisées pendant de longues périodes de temps ce qui nécessite une excellente stabilité non seulement au stockage mais également dans de larges domaines de température. En d'autres termes, ces compositions ne doivent pas donner naissance, à des troubles provoqués par l'instabilité de certains de leurs ingrédients, notamment du ou des agents tensio-actifs employés.

La présente invention se propose de fournir une nouvelle composition cosmétique de nettoyage, notamment une lotion de démaquillage des yeux dont l'agent nettoyant est constitué par un mélange d'agents tensioactifs, ce mélange ayant seul permis d'obtenir des compositions présentant l'ensemble des propriétés requises pour ce type de produit, c'est à dire un excellent pouvoir démaquillant, un bon confort cosmétique, une bonne innocuité en test oculaire et une excellente conservation dans le temps à différentes températures.

La présente invention a pour objet, à titre de produit industriel nouveau, une composition cosmétique pour le nettoyage, notamment pour le démaquillage des yeux, contenant en solution aqueuse, en plus des ingrédients usuels pour ce type de composition, un agent nettoyant constitué par un mélange d'agents tensio-actifs dans les proportions suivantes:

(i) — de 0,1 à 2% en poids en matière active d'un alkyléther de glucoside correspondant à la formule:

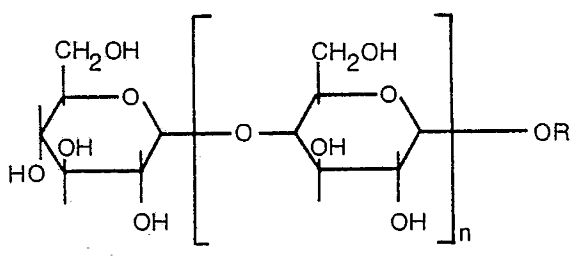

(I)

dans laquelle:

R désigne un radical alkyle ayant de 8 à 12 atomes de carbone, n est égal à 0, 1, 2, 3, 4 ou 5;

(ii) — de 0,1 à 2% en poids en matière active d'un amphotère de formule:

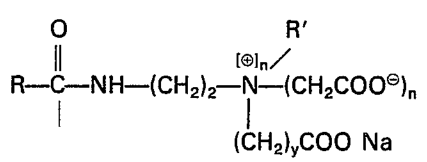

(II)

dans laquelle:

n est 0 ou 1

y est 1 ou 2

R représente une chaîne grasse ayant de 7 à 17 atomes de carbone, de préférence 11 atomes de carbone, et

R' représente $-CH_2-CH_2OH$ ou $-(CH_2)_2-O-CH_2)_2-COONa$,

éventuellement en mélange avec un alkyl sulfate oxyéthyléné de formule:

$$C_{13}H_{27}-(OCH_2CH_2)_m-OSO_3Na \qquad (III)$$

dans laquelle:

m est 1 à 4, et

(iii) de 0,3 à 5% en poids en matière active d'un composé pris dans le groupe constitué par:

(a) le monolaurate ou le monooléate de Sorbitan polyoxyéthyléné à l'aide de 20 moles d'oxyde d'éthylène,

(b) le monolaurate ou le monooléate de glycérol polyoxyéthyléné à l'acide de 20 moles d'oxyde d'éthylène, et

(c) un composé non-ionique correspondant à la formule:

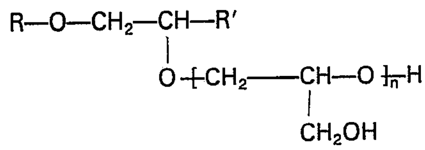

(IV)

dans laquelle:

R représente une chaîne grasse de 8 à 10 atomes de carbone,

R' représente une chaîne grasse ayant de 10 à 16 atomes de carbone, et

n est compris entre 10 et 12, le rapport

$$\frac{n}{R+R'}$$

étant compris entre 0,4 et 0,6.

Les différents essais effectués ont en effet permis de montrer que seule l'association de ces trois agents tensio-actifs, dans les proportions indiquées, était susceptible de conduire à l'ensemble des propriétés recherchées. La présence du monolaurate ou du mono-oléate de sorbitan, ou de glycérol, polyoxyéthyléné s'est avéré indispensable en vue d'obtenir une bonne stabilité de la composition dans le temps, sand que l'on observe l'apparition d'un trouble irréversible lorsque la composition est soumise à une température inférieure à 10°C, puis ramenée à température ambiante.

En effet, lorsque l'on utilise uniquement comme agent nettoyant l'association d'un alkyléther de glucoside de formule (I) et d'un amphotère de formule (II) soit seul, soit en mélange avec le composé de formule (III), la composition nettoyante, si elle présente après sa préparation de bonnes propriétés démaquillante et d'innocuité, manque par contre rapidement de stabilité ce qui, dès lors, la rend inutilisable.

Selon une mode de réalisation préféré de l'invention, l'agent nettoyant est constitué de:

(i) — 0,2 à 1,5% en poids en matière active de l'alkyléther de glucoside de formule (I),

(ii) — 0,2 à 1,5% en poids en matière active de l'amphotère de formule (II) soit seul soit en mélange avec le composé de formule (III)

(iii) — 0,5 à 3% en poids en matière active de monolaurate ou de monooléate de sorbitan, ou de glycérol, polyoxyéthyléné à l'aide de 20 moles d'oxyde d'éthylène ou d'un composé non-ionique de formule (IV).

Parmi les alkyléthers de glucoside de formule (I) on peut en particulier mentionner celui dans lequel le radical R représente un radical $C_{12}H_{25}$ et n=5 ou encore ceux dans lesquels le radical R représente un mélange $C_8$—$C_{10}$ (50/50) et n est égal à 0 ainsi que ceux dans lesquels R a la même signification et n est 1 à 4, notamment le produit commercial vendu par la Société ROHM et HAAS sous la marque "TRITON CG. 110-30".

Parmi les amphotères de formule (II) on peut en particulier mentionner ceux vendus par la Société MIRANOL CHEMICAL Company Inc. sous les marques "MIRANOL C2M Conc" et "MIRANOL C2M SF Conc", "MIRANOL 2 MCT MOD", "MIRANOL 2 MHT MOD" et "MIRANOL MHT".

Parmi les composés de formule (III) éventuellement associés aux composés de formule (II) on peut citer ceux vendus par la Société MIRANOL CHEMICAL Company Inc. sous les marques "MIRANOL 2 MCT MOD", "MIRANOL BT" "MIRANOL 2 MHT MOD" et "MIRANOL MHT".

Le monolaurate de sorbitan polyoxyéthyléné à l'aide de 20 moles d'oxyde d'éthylène est plus connu sous la marque "TWEEN 20" et le mono-oléate de sorbitan polyoxyéthyléné à l'aide de 20 moles d'oxyde d'éthylène sous la marque "TWEEN 80".

Bien que le mono-oléate de glycérol polyoxyéthyléné à l'aide de 20 moles d'oxyde d'éthylène conduise à des résultats satisfaisants, on préfère selon l'invention utiliser le monlaurate de glycérol polyoxyéthyléné à l'aide de 20 moles d'oxyde d'éthylène vendu sous la marque "TAGAT.L.2" par la Société GOLDSCHMIDT.

Parmi les composés non-ioniques de formule (IV), on peut en particulier citer ceux décrits dans le brevet belge No. 885.269 notamment celui dans lequel le radical R représente $C_8H_{17}$, le radical R' représente $C_{14}H_{29}$ et n=12.

Le véhicule des compositions de nettoyage selon l'invention est soit de l'eau déminéralisée stérile soit

une eau florale telle que de l'eau de rose, de l'eau de bleuet, de l'eau de camomille, ou de l'eau de tilleul.

Les autres ingrédients de la composition de nettoyage sont essentiellement un agent conservateur qui peut être par exemple d l'éthyl mercurithiosalicylate de sodium, un sel de chlorhexidine tel que le digulconate, le diacétate et le dichlorhydrate, un sel de phénylmercure tel que le nitrate de phénylmercure, un mélange constitué de 30% de benzoate de sodium et de 70% de monochloracétamide, un composé correspondant à la formule suivante:

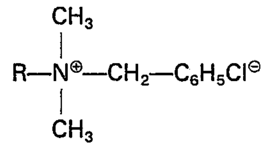

dans laquelle:

R représente un radical alkyle ayant de 12 à 18 atomes de carbone ou un mélange de tels radicaux alkyles tels que les mélanges $C_{12}$—$C_{14}$ et $C_{14}$—$C_{16}$.

L'agent conservateur des compositions selon l'invention est généralement compris entre 0,002 et 0,8% et de préférence entre 0,02 et 0,5%.

Le pH des compositions selon l'invention est généralement compris entre 6,5 et 7,5 et de préférence entre 7 et 7,2 et obtenu à l'aide d'un agent tampon tel que par exemple un tampon phosphate (hydrogénophosphate de dipotassium/dihydrogénophosphate de potassium).

Les compositions de nettoyage selon l'invention peuvent également contenir un polymère non ionique ou anionique. On a constaté de façon tout à fait surprenante que lorsque l'on utilisait dans les compositions de nettoyage selon l'invention un polymère non ionique ou anionique dans une proportion comprise entre 0,1 et 3%, il était possible de réduire de façon substantielle les concentrations des trois agents tensio-actifs sans pour autant influer sur les propriétés de la composition, notamment sur son pouvoir nettoyant.

Parmi les polymères non ioniques qui peuvent être utilisés dans les compositions selon l'invention on peut citer:

(i) — Les poly β-alanines décrites dans la demande de brevet belge No. 208.516

Ces polymères comportant de 50 à 100% de motifs répétitifs de formule:

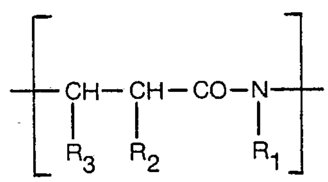

et de 0 à 50% de motifs répétitifs du type polyacrylamide correspondant à la formule suivante:

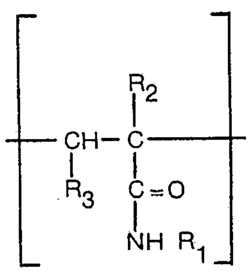

dans lesquels $R_1$ représente un atome d'hydrogène, ou un radical pris dans le groupe constitué par les radicaux suivants:

(i)

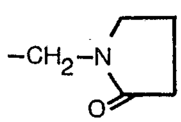,

(ii) —$CH_2OH$,

(iii) —($CH_2n'$—$CH_3$, n'étant 0 ou un nombre entier de 1 à 11 et

(iv) —($CH_2$—$CH_2$—O $)_m$—H, m étant compris entre 1 et 10,

et $R_2$ et $R_3$ représentent un atome d'hydrogène ou un radical méthyle.

Ces polymères sont préparés par polymérisation de l'acrylamide, comme décrit dans le brevet américain 4.082.730. Ces polymères ont de préférence un poids molécule compris entre 500 et 200 000 et plus particulièrement entre 2000 et 100 000,

(ii) — la polyvinylpyrrolidone ayant un poids moléculaire compris entre 10.000 et 400.000.

(iii) — l'hydroxyéthylcellulose, comme par exemple le produit vendu sous la marque "NATROSOL 250" par la Société HERCULES.

(iv) — les dérivés hydroxypropylés de gomme guar, en particulier le produit vendu sous la marque "JAGUAR H P-8 par la Société MEYHALL. Parmi les polymères anioniques, on peut en particulier citer:

(i) — le copoolymère d'éther méthyl vinylique et d'acide maléique obtenu par hydryolyse du copolymère anhydride correspondant, tel que celui vendu par la Société GAF Corp. sous la marque "RESINE GANTREZ AN 119"

(ii) — le polyméthacrylate de sodium tel que celui vendu par la Société VANDERBILT sous la marque "DARVAN 7", et

(iii) — les polymères d'acide acrylique tels que ceux vendus par la Société ALLIED COLLOIDS sous la marque "VERSICOL $E_5$" (PM = 2.500) ou "VERSICOL $E_{11}$ " (PM = 230.000).

Les compositions selon l'invention peuvent également contenir d'autres adjuvants conventionnels tels que par exemple des agents humectants, des agents adoucissants, des parfums ou des colorants, ceux-ci devant bien entendu présenter la particularité d'être stables au sein de la composition et ne pas provoquer d'irritation ou de picotement de la muqueuse oculaire.

Parmi les agents humectants on peut en particulier citer l'hexylèneglycol et le polyéthylèneglycol 600.

Parmi les agents adoucissants on peut en particulier mentionner l'allontoïne et l'azulène.

On va maintenant donner à titre d'illustration et sans aucun caractère limitatif plusieurs modes de réalisation des compositions de nettoyage selon l'invention.

## Exemple 1

On prépare selon l'invention une lotion démaquillante pour les yeux en procédant au mélange des ingrédients suivants:

| | |
|---|---|
| Triton CG.110-30® (à 30% en matière active) | 1 g |
| Miranol C2M® | 1 g |
| Tween 20® | 1 g |
| Allantoïne | 0,05 g |
| Hexylène glycol | 2 g |
| Dihydrogénophosphate de potassium | 0,1 g |
| Hydrogénophosphate dipotassique | 0,3 g |
| Chlorure de benzalkonium | 0,25 g |
| Eau de tilleul | 10 g |
| Parfum | 0,1 g |
| Eau déminéralisée stérile qsp | 100 g |

Cette solution présente un pH de 7,2 environ.

## Exemple 2

On prépare selon l'invention une lotion démaquillante pour les yeux en procédant au mélange des ingrédients suivants:

| | |
|---|---|
| Triton CG.110-30® (à 30% en matière active) | 0,5 g |
| Miranol C2M® | 0,5 g |
| Tween 20® | 0,5 g |
| Polyβ-alanine non ionique | 0,85 g |
| Allantoïne | 0,05 g |

| | | |
|---|---|---|
| Hexylène glycol | 2 | g |
| Dihydrogénophosphate de potassium | 0,1 | g |
| Hydrogénophosphate dipotassique | 0,3 | g |
| Ethylmercurithiosalicylate de sodium | 0,003 | g |
| Eau de bleuet | 10 | g |
| Parfum | 0,1 | g |
| Eau déminéralisée stérile qsp | 100 | g |

Cette solution présente un pH de 7,1 environ

Dans cet exemple la présence de la polyβ-alanine permet par rapport à l'exemple 1 de réduire de moitié la concentration des trois agents tensio-actifs sans constater pour autant une modification des propriétés de la lotion notamment de son pouvoir démaquillant.

Example 3

On prépare selon l'invention une composition démaquillante pour les yeux en procédant au mélange des ingrédients suivants:

| | | |
|---|---|---|
| Lauryl éther de glucoside (formule (I) dans laquelle R=Lauryl et n=5) | 0,7 | g |
| Miranol 2MHT MOD® (modifié) | 0,5 | g |
| Monolaurate de glycérol à 20 moles d'oxyde d'éthylène (TAGAT L2®) | 0,6 | g |
| Versicol E5-E11® | 0,3 | g |
| Dihydrogènophosphate de K | 0,1 | g |
| Hydrogènophosphate de K | 0,3 | g |
| Ethyl mercurithiosalicylate de sodium | 0,003 | g |
| Parfum | 0,1 | g |
| Eau qsp | 100 | g |

Cette solution présente un pH de 7 environ

Exemple 4

On prépare selon l'invention une composition démaquillante pour les yeux en procédant au mélange des ingrédients suivants:

| | | |
|---|---|---|
| Alcoylglucoside de formule (I) dans laquelle $R=C_8/C_{10}$ (1/1) et n=0 | 1,2 | g |
| Miranol MHT® | 0,9 | g |
| Tween 80® | 0,7 | g |
| Dihydrogènophosphate de K | 0,1 | g |
| Hydrogènophosphate de K | 0,3 | g |
| Ethylmercurithiosalicylate de sodium | 0,003 | g |

| | | | |
|---|---|---|---|
| Parfum | | 0,1 | g |
| Eau | qsp | 100 | g |

Cette solution présente un pH de 7,1 environ.

Exemple 5

On prépare selon l'invention une lotion démaquillante pour les yeux en procédant au mélange des ingrédients suivants:

| | | | |
|---|---|---|---|
| Triton CG.110-30®. (à 30% en matière active) | | 1 | g |
| Miranol C2M® | | 1 | g |
| Composé non-ionique de formule (IV) dans laquelle: $R=C_8H_{17}$, $R'=C_{14}H_{29}$ et n=12 | | 1,5 | g |
| Allantoïne | | 0,05 | g |
| Hexylène glycol | | 2 | g |
| Dihydrogénophosphate de K | | 0,1 | g |
| Hydrogénophosphate de K | | 0,3 | g |
| Chlorure de Benzalkonium | | 0,25 | g |
| Parfum | | 0,1 | g |
| Eau | qsp | 100 | g |

Cette lotion présente un pH de 7 environ.

**Revendications**

1. Composition cosmétique pour le nettoyage, notamment pour le démaquillage des yeux, caractérisée par le fait qu'elle contient, en solution aqueuse, en plus des ingrédients usuels pour ce type de composition, un agent nettoyant constitué par un mélange d'agents tensio-actifs dans les proportions suivantes:

(i) — de 0,1 à 2% en poids en matière active d'un alkyléther de glucoside correspondant à la formule:

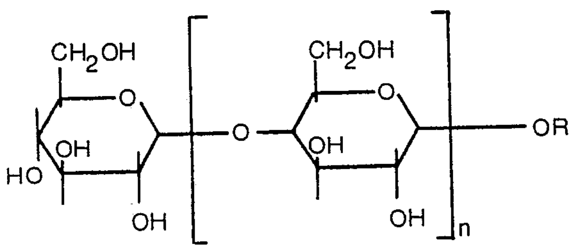

(I)

dans laquelle:

R désigne un radical alkyle ayant de 8 à 12 atomes de carbone, n est égal à 0, 1, 2, 3, 4 ou 5;

(ii) — de 0,1 à 2% en poids en matière active d'un amphotère de formule:

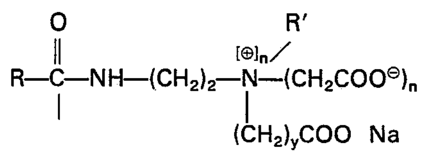

(II)

dans laquelle:

n est 0 ou 1

y est 1 ou 2

R représente une chaîne grasse ayant de 7 à 17 atomes de carbone, de préférence 11 atomes de carbone, et

R' représente —$CH_2$—$CH_2OH$ ou —$(CH_2)_2$—O—$(CH_2)_2$—COONa, éventuellement en mélange avec un alkyl sulfate oxyéthyléné de formule:

$$C_{13}H_{27}—(OCH_2CH_2)_m—OSO_3Na \quad \text{(III)}$$

dans laquelle:

m est 1 à 4, et

(iii) de 0,3 à 5% en poids en matière active d'un composé suivant:

(a) le monolaurate ou le monooléate de Sorbitan polyoxyéthyléné à l'aide de 20 moles d'oxyde d'éthylène,

(b) le monolaurate ou le monooléate de glycérol polyoxyéthyléné à l'acide de 20 moles d'oxyde d'éthylène, et

(c) un composé non-ionique correspondant à la formule:

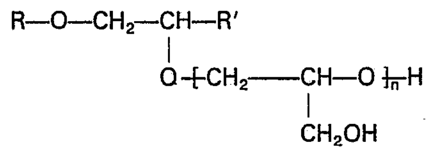

(IV)

dans laquelle:

R représente une chaîne grasse de 8 à 10 atomes de carbone,

R' représente une chaîne grasse ayant de 10 à 16 atomes de carbone, et

n est compris entre 10 et 12, le rapport

$$\frac{n}{R+R'}$$

étant compris entre 0,4 et 0,6.

2. Composition selon la revendication 1, caractérisée par le fait que l'agent nettoyant est de préférence constitué de:

(i) — 0,2 à 1,5% en poids en matière active de l'alkyléther de glucoside de formule (I),

(ii) — 0,2 à 1,5% en poids en matière active de l'amphotère de formule (II) soit seul soit en mélange avec le composé de formule (III)

(iii) — 0,5 à 3% en poids en matière active de monolaurate ou de monooléate de sorbitan, ou de glycérol, polyoxyéthyléné à l'aide de 20 moles d'oxyde d'éthylène ou d'un composé non-ionique de formule (IV).

3. Composition selon l'une quelconque des revendications 1 et 2, caractérisée par le fait qu'elle contient en outre un agent conservateur selectioné parmi l'éthyl mercurithiosalicylate de sodium, un sel de chlorhexidine, un sel de phénylmercure, un mélange constitué de 30% de benzoate de sodium et de 70% de monochloroacétamide, un composé correspondant à la formule suivante:

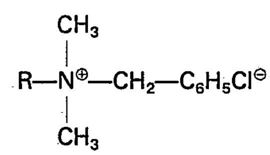

dans laquelle:

R représente un radical alkyle ayant de 12 à 18 atomes de carbone ou un mélange de tels radicaux alkyles.

4. Composition selon la revendication 3, caractérisée par le fait que l'agent conservateur est présent à une concentration comprise en 0,002 et 0,8% et de préférence entre 0,02 et 0,5%.

5. Composition selon l'une quelconque des revendications précédentes caractérisée par le fait qu'elle présente un pH compris entre 6,5 et 7,5 et de préférence entre 7 et 7,2.

6. Composition selon l'une quelconque des revendications précédentes caractérisée par le fait qu'elle contient en outre au moins un polymère non ionique ou anionique.

7. Composition selon la revendication 6, caractérisée par le fait que le polymère non ionique ou anionique est présent dans une proportion comprise entr 0,1 et 3% par rapport au poids total de la composition.

8. Composition selon l'une quelconque des revendications 6 et 7, caractérisée par le fait que le polymère non ionique est selectioné parmi:

les poly β-alanines, la polyvinylpyrrolidone, l'hydroxyéthylcellulose et les dérivés de gomme de guar.

9. Composition selon l'une quelconque des revendications 6 et 7, caractérisée par le fait que le

polymère anionique est selectioné parmi
le copolymère d'éther méthyl vinylique et d'acide maléique, le polyméthacrylate de sodium et les polymères d'acid e acrylique.

10. Composition selon l'une quelconque des revendications précédentes, caractérisée par le fait qu'elle contient en outre au moins un agent humectant, un agent adoucissant, un parfum ou un colorant.

**Patentansprüche**

1. Kosmetisches Reinigungsmittel, insbesondere zum Abschminken der Augen, dadurch gekennzeichnet, daß es in einer wäßrigen Lösung zusätzlich zu für eine solche Zusammensetzung üblichen Bestandteilen ein Reinigungsmittel enthält, das aus einer Mischung oberflächenaktiver Mittel in den folgenden Anteilen besteht:

(i) 0,1 bis 2 Gew.-% eines Alkylethers eines Glukosids der folgenden Formel als Wirkstoff:

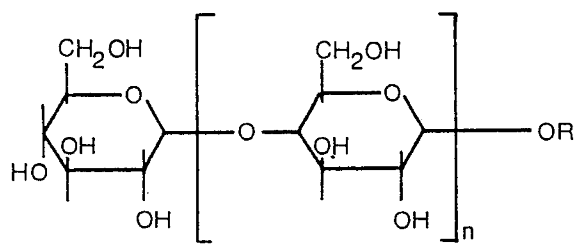

(I)

worin:
R ein Alkylrest mit 8 bis 12 Kohlenstoffatomen darstellt, und n gleich 0, 1, 2, 3, 4 oder 5 ist;

(ii) 0,1 bis 2 Gew.-% eines amphoteren Wirkstoffes der Formel:

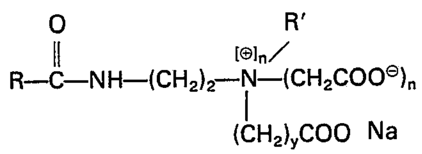

(II)

worin:
n 0 oder 1 ist, y 1 oder 2 ist,
R einen Fettrest mit 7 bis 17 Kohlenstoffatomen, vorzugsweise 11 Kohlenstoffatomen, bedeutet, und
R' $-CH_2-CH_2OH$ oder $-(CH_2)_2-O-(CH_2)_2-COONa$,
bedeutet,
gegebenenfalls in Mischung mit einem Alkyloxyethylensulfat der Formel:

$$C_{13}H_{27}-(OCH_2CH_2)_m-OSO_3Na \quad (III)$$

worin:
m 1 bis 4 ist, und

(iii) 0,3 bis 5 Gew.-% eines Wirkstoffes der folgenden Zusammensetzung:
(a) das Monolaurat oder das Monooleat von Polyoxyethylensorbitan mit 20 Mol Ethylenoxid,
(b) das Monolaurat oder das Monooleat von Polyoxyethylenglyzerin mit 20 Mol Ethylenoxid, und
(c) eine nicht-ionische Zusammensetzung der Formel:

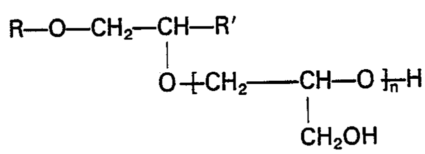

(IV)

worin:
R eine Fettkette mit 8 bis 10 Kohlenstoffatomen bedeutet,
R' eine Fettkette mit 10 bis 16 Kohlenstoffatomen bedeutet, und
n im Berich zwischen 10 und 12 liegt, und das Verhältnis

$$\frac{n}{R+R'}$$

zwischen 0,4 und 0,6 ist.

22. Zusammensetzung nach Anspruch 1, dadurch gekennzeichnet, daß das Reinigungsmittel vorzugsweise zusammengesetzt ist aus:

(i) 0,2 bis 1,5 Gew.-% eines Alkylethers eines Glukosids der Formel (I) als Wirkstoff;

(ii) 0,2 bis 1,5 Gew.-% eines amphoteren Wirkstoffs der Formel (II), entweder allein oder in Mischung mit der Verbindung der Formel (III),

(iii) 0,5 bis 3 Gew.-% eines Wirkstoffs aus dem Monolaurat oder dem Monooleat von Sorbitan oder von Glyzerin, mit Hilfe von 20 Mol Ethylenoxid polyoxyethyliert, oder aus einer nicht-ionischen Verbindung der Formel (IV).

3. Zusammensetzung nach einem der Ansprüche 1 und 2, dadurch gekennzeichnet, daß sie zusätzlich ein Konservierungsmittel enthält, ausgewählt aus Natriummercurithiosalicylat, einem Salz von Chlorhexidin, einem Salz von Phenylquecksilber, einer Mischung aus 30% Natriumbenzoat und 70% Monochloracetamid, einer Verbindung der folgenden Formel:

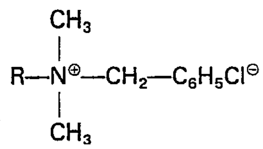

worin:

R eine Alkylrest mit 12 bis 18 Kohlenstoffatomen oder eine Mischung dieser Alkylreste darstellt.

4. Zusammensetzung nach Anspruch 3, dadurch gekennzeichnet, daß das Konservierungsmittel in einer Konzentration im Bereich von 0,002 und 0,8%, vorzugsweise zwischen 0,02 und 0,5% vorliegt.

5. Zusammensetzung nach einem der vorhergehenden Ansprüche, dadurch gekennzeichnet, daß sie einen pH-Wert im Bereich zwischen 6,5 und 7,5, vorzugsweise zwischen 7 und 7,2, aufweist.

6. Zusammensetzung nach einem der vorhergehenden Ansprüche, dadurch gekennzeichnet, daß sie zusätzlich mindestens ein nicht-ionisches oder anionisches Polymeres enthält.

7. Zusammensetzung nach Anspruch 6, dadurch gekennzeichnet, daß das nichtionische oder anionische Polymere in einem Anteil im Bereich zwischen 0,1 und 3%, bezogen auf das Gesamtgewicht der Zusammensetzung, vorliegt.

8. Zusammensetzung nach einem der Ansprüche 6 und 7, dadurch gekennzeichnet, daß das nicht-ionische Polymere ausgewählt ist aus:

Poly-β-alanine, Polyvinylpyrrolidon, Hydroxyethylcellulose und Derivate von Guargummi.

9. Zusammensetzung nach einem der Ansprüche 6 und 7, dadurch gekennzeichnet, daß das anionische Polymere ausgewählt ist aus:

Copolymere von Methylvinylether und Maleinsäure, Natriumpolymethacrylat und Acrylsäurepolymere.

10. Zusammensetzung nach einem der vorhergehenden Ansprüche, dadurch gekennzeichnet, daß sie zusätzlich mindestens ein Anfeuchtungsmittel, einen Weichmacher, ein Parfüm oder einen Farbstoff enthält.

**Claims**

1. Cosmetic composition for cleansing, in particular for removing make up from the eyes, characterized in that it contains, in aqueous solution, in addition to the usual ingredients for this type of copositions, a cleansing agent consisting of a mixture of surfactants in the following proportions:

(i) from 0.1 to 2% by weight of active substance of a glucoside alkyl ether corresponding to the formula:

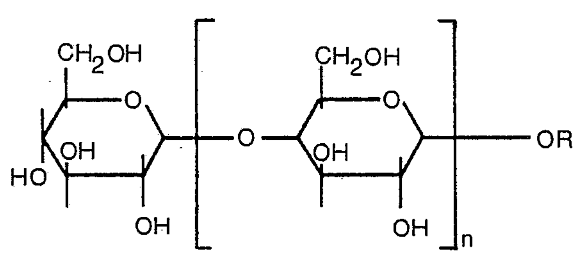

(I)

in which:

R denotes an alkyl radical having from 8 to 12 carbon atoms, and n equals 0, 1, 2, 3, 4 or 5;

(ii) from 0.1 to 2% by weight of active substance of an amphoteric compound of formula:

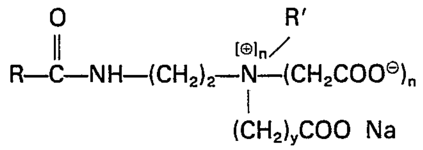 (II)

in which:

n is 0 or 1,

y is 1 or 2,

R denotes a fatty chain having from 7 to 17 carbon atoms, preferably 11 carbon atoms, and

R′ denotes $-CH_2-CH_2OH$ or $-(CH_2)_2-O-(CH_2)_2-COONa$,

optionally mixed with an oxyethylenated alkyl sulphate of formula:

$$C_{13}H_{27}-(OCH_2CH_2)_m-OSO_3Na \qquad (III)$$

in which:

m is 1 to 4, and

(iii) from 0.3 to 5% by weight of active substance of one of the following compounds:

(a) sorbitan monolaurate or monooleate polyoxyethylenated with 20 moles of ethylene oxide,

(b) glycerol monolaurate or monooleated polyoxyethylenated with 20 moles of ethylene oxide, and

(c) a nonionic compound corresponding to the formula:

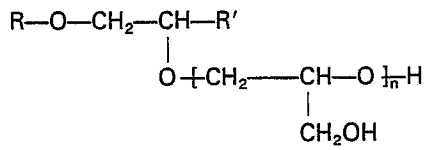 (IV)

in which:

R denotes a fatty chain of 8 to 10 carbon atoms,

R′ denotes a fatty chain having from 10 to 16 carbon atoms, and

n is between 10 and 12, the ratio

$$\frac{n}{R+R}$$

being between 0.4 and 0.6.

2. Composition according to Claim 1, characterized in that the cleansing agent preferably consists of:

(i) 0.2 to 1.5% by weight of active substance of the glucoside alkyl ether of formula (I),

(ii) 0.2 to 1.5% by weight of active substance of the amphoteric compound of the formula (II), either alone or mixed with the compound of formula (III), and

(iii) 0.5 to 3% by weight of active substance of sorbitan monolaurate or monooleate or glycerol monolaurate or monooleate, polyoxyethylenated with 20 moles of ethylene oxide, or of a nonionic compound of formula (IV).

3. Composition according to either of Claims 1 and 2, characterized in that it contains, in addition, a preservative selected from sodium ethylmercurythiosalicylate, a chlorhexidine salt, a phenylmercury salt, a mixture consisting of 30% of sodium benzoate and 70% of monochloroacetamide, and a compound corresponding to the following formula:

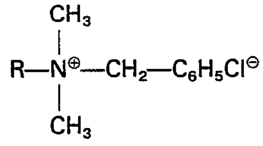

in which:

R denotes an alkyl radical having from 12 to 18 carbon atoms or a mixture of such alkyl radicals.

4. Composition according to Claim 3, characterized in that the preservative is present at a concentration of between 0.002 and 0.8%, preferably between 0.02 and 0.5%.

5. Composition according to any one of the preceding claims, characterized in that it has a pH of between 6.5 and 7.5, and preferably between 7 and 7.2.

6. Composition according to any one of the preceding claims, characterized in that it contains, in addition, at least one nonionic or anionic polymer.

7. Composition according to Claim 6, characterized in that the nonionic or anionic polymer is present in a proportion of between 0.1 and 3%, based on the total weight of the composition.

8. Composition according to either of Claims 6 and 7, characterized in that the nonionic polymer is selected from:

poly β-alanines, polyvinylpyrrolidone, hydroxyethylcellulose and guar gum derivatives.

9. Composition according to either of Claims 6 and 7, characterized in that the anionic polymer is selected from:

the copolymer of methyl vinyl ether and maleic acid, sodium polymethacrylate and acrylic acid polymers.

10. Composition according to any one of the preceding claims, characterized in that it contains, in addition, at least a humectant, an emollient, a perfume or a colouring.